# EUROPÄISCHE PATENTSCHRIFT

(11) **EP 2 931 187 B1**
(45) Veröffentlichungstag und Bekanntmachung des Hinweises auf die Patenterteilung: **30.08.2017**
(21) Anmeldenummer: 13807892.8
(22) Anmeldetag: 05.12.2013
(51) Int. Cl.: A61F 2/95, A61F 2/966

(54) **STENTAPPLIKATOR**
STENT APPLICATOR
APPLICATEUR DE STENT

(30) Priorität: 11.12.2012 CH 27712012
(43) Veröffentlichungstag der Anmeldung: 21.10.2015
(73) Patentinhaber: Carag AG, 6340 Baar (CH)
(72) Erfinder: HUMMEN, Jörg, CH-6402 Merlischachen (CH); LIMACHER, Kuno, CH-6312 Steinhausen (CH); RÖTHLIN, Cyrill, CH-6331 Hünenberg (CH); STEINER, Claudio, CH-6340 Baar (CH); WIDMER, Beat, CH-6005 Luzern (CH); DE PABLO PEÑA, Albora, CH-8037 Zürich (CH); BERNHARD, Jerôme, CH-8003 Zürich (CH); SIDLER, Christoph, CH-6030 Ebikon (CH)
(74) Vertreter: Clerc, Natalia
(86) Internationale Anmeldenummer: PCT/CH2013/000215
(87) Internationale Veröffentlichungsnummer: WO 2014/089715

(56) Entgegenhaltungen:
- EP-A2- 1 208 816
- WO-A1-00/18330
- WO-A1-2008/008642
- WO-A1-2009/011866
- WO-A1-2010/130443
- US-A1- 2006 030 923
- US-A1- 2006 184 226
- US-A1- 2008 132 989
- US-A1- 2011 276 121

## Beschreibung

### TECHNISCHES GEBIET

Die vorliegende Erfindung betrifft einen Stentapplikator, d.h. ein Zuführungssystem zum Zuführen eines Stents in einen Körper eines Patienten gemäss Oberbegriff des Patentanspruchs 1 sowie einen Katheterkopf.

### STAND DER TECHNIK

Stents und Applikatoren zur Einführung und Entfaltung von Stents sind im Stand der Technik in den verschiedensten Ausführungsformen bekannt. Ein Stent ist üblicherweise eine zylinderförmige Prothese, welche in ein Gefäss oder ein Lumen eines Patienten eingeführt und dort radial expandiert wird. Stents werden in den verschiedensten Bereichen eingesetzt, beispielsweise als Herzklappenstents, Koronarstents oder Peripherstents. In bevorzugten Ausführungsformen sind die Stents selbstexpandierend ausgebildet, insbesondere mittels Federkraft. Es sind beispielsweise auch ballonexpandierte Stents bekannt.

Als Zuführsystem (delivery system), auch Stentapplikator genannt, werden üblicherweise Katheter eingesetzt, welche eine speziell ausgebildete Katheterspitze aufweisen. Diese Spitze umschliesst den zusammengepressten Stent und ist so ausgebildet, dass sie nach Massgabe des Arztes den Stent an der gewünschten Stelle freigeben kann. Es ist notwendig, dass dieser Katheter und insbesondere diese Spitze einen möglichst kleinen Aussendurchmesser aufweist.

Sehr verbreitet sind Stentapplikatoren, welche eine Mechanik mit Zug- und Druckstangen aufweisen.

Im Stand der Technik sind jedoch auch Stentapplikatoren bekannt, welche mit einem Fluid betätigt werden, um den Stent freizusetzen. Beispiele hierfür sind EP 0 746 375, EP 1 565 227, WO 2005/115524, EP 0 705 578, WO 2008/153765, US 6 113 608, EP 1 208 816, US 6 056 759, US 6 027 4474, WO 2006/096229 und US 2006/030923. WO 2009/011866 offenbart ferner ein Zuführungssystem für Stents mit einem doppeltwirkenden Zylinder, welcher im Manipulationsbesteck des Katheters angeordnet ist. Dieses System soll das Entfalten des Stents erleichtern.

Diese Stentapplikatoren ermöglichen ein Freisetzen eines Stents am gewünschten Ort innerhalb eines Körpergefässes. Sie sind jedoch nicht geeignet, einen teilweise oder ganz freigesetzten Stent wieder zu umhüllen, um ihn neu zu platzieren oder um ihn wieder ganz aus dem Körper zu entfernen.

Eine derartige neue Platzierung oder Entfernung des Stents ist jedoch oft erwünscht. Beispielsweise kann es während der Implantation am schlagenden Herzen zu einer Dislokation des Stents kommen, so dass nochmals neu platziert werden muss.

### DARSTELLUNG DER ERFINDUNG

Es ist deshalb eine Aufgabe der Erfindung, ein Zuführungssystem für einen Stent zu schaffen, welches nicht nur ein Freisetzen, sondern auch ein erneutes Umhüllen des Stents ermöglicht.

Diese Aufgabe löst ein Zuführungssystem mit den Merkmalen des Patentanspruchs 1.

Das erfindungsgemässe Zuführungssystem zum Zuführen eines Stents in einen Körper eines Patienten, beispielsweise in ein Gefäss oder ein Lumen, umfasst einen flexiblen Katheterschlauch mit einem proximalen und einem distalen Ende zum Zuführen des Stents an einen vorgegebenen Ort im Körpergefäss und eine Katheterspitze mit einer Längsrichtung, welche am distalen Ende des Katheterschlauchs angeordnet ist. Die Katheterspitze weist eine Stentkammer zur Aufnahme des Stents auf. Die Katheterspitze weist eine erste Druckkammer auf. Im Katheterschlauch verläuft ein erster Fluidkanal vom proximalen Ende bis zur ersten Druckkammer der Katheterspitze. Ein Volumen der ersten Druckkammer ist durch Zuführen eines ersten Fluids vom ersten Fluidkanal zur ersten Druckkammer vergrösserbar, wobei der Stent dadurch aus der Stentkammer am vorgegebenen Ort im Körpergefäss freisetzbar ist. Erfindungsgemäss weist die Katheterspitze eine zweite Druckkammer auf. Im Katheterschlauch verläuft ein zweiter Fluidkanal vom proximalen Ende bis zur zweiten Druckkammer. Ein Volumen der zweiten Druckkammer ist durch Zuführen eines zweiten Fluids vom zweiten Fluidkanal zur zweiten Druckkammer vergrösserbar, wodurch ein teilweise freigesetzter Stent vom vorgegebenen Ort im Körpergefäss in die Stentkammer rückführbar ist.

"Proximal" und "hinten" bedeuten in diesem Text dem Arzt zugewandt und dem Patienten abgewandt. "Distal" und "vorne" bedeuten in diesem Text dem Arzt abgewandt und dem Patienten zugewandt.

Der erfindungsgemässe Stentapplikator ermöglicht nicht nur ein zielgenaues Platzieren eines Stents in einem Gefäss eines Patienten, sondern auch dessen Rückholung in situ. Der Stent kann im lediglich teilweise freigesetzten Stadium stets zurückgeholt werden. Eine Rückholung und erneute Umhüllung eines vollständig freigesetzten Stents hängt von der Form und Art des Stents und seiner Verbindung zum Stentapplikator ab, ist jedoch mit dem erfindungsgemässen Mechanismus auch möglich.

Dank des erfindungsgemässen Systems lässt sich der Stent genau an der Stelle platzieren, an welcher er bereits in umhüllter Stellung gelegen hat. Er wird nicht, wie im Stand der Technik üblich, nach vorne geschoben. Vielmehr wird seine Umhüllung nach hinten weg gezogen. Dies ermöglicht eine genaue Positionierung des Stents innerhalb der Körperöffnung bzw. des Körperkanals.

In bevorzugten Ausführungsformen weist das Zuführsystem einen doppelt wirkenden Hydraulikzylinder auf, welcher den Stent freisetzt und wieder einhüllt. Vorzugsweise sind die zwei Druckkammern in der Katheterspitze nicht miteinander verbunden, d.h. sie tauschen keine Fluide aus. Vorzugsweise tauscht das gesamte System die zwei Fluide nicht untereinander aus.

Der Katheterschlauch ist vorzugsweise als Multilumenkatheterschlauch ausgebildet, welcher mindestens zwei voneinander getrennte Leitungen sowie vorzugsweise ein Lumen für einen Führungsdraht aufweist. Dieser Schlauch dient zugleich als flexible Kolbenstange für die Verschiebung des mindestens einen in der Katheterspitze angeordneten Katheterkolbens bzw. er wirkt selber als Kolben.

In bevorzugten Ausführungsformen bewegt sich der Katheterschlauch während des Freisetzens und Wiedereinhüllen des Stents nicht mehr, nachdem er die Katheterspitze an den vorgegebenen Ort im Körpergefäss gebracht hat. In anderen Ausführungsformen bleibt nur ein innerer Teil des Katheterschlauchs immobil und ein äusserer Teil bewegt sich während des Freisetzens und/oder während des Wiedereinhüllens des Stents.

In bevorzugten Ausführungsformen weist die Katheterspitze eine erste Hülse auf, in welcher die erste Druckkammer angeordnet ist, und eine zweite Hülse, in welcher die zweite Druckkammer angeordnet ist.

Vorzugsweise weist der Katheterschlauch ein Führungslumen zur Durchführung eines Führungsdrahtes auf, wobei die erste Hülse und die zweite Hülse relativ zu diesem Führungslumen verschiebbar sind und wobei das Führungslumen in seiner Lage bezüglich des vorgegebenen Ortes unverändert bleibt und sich erste und zweite Hülse relativ zum vorgegebenen Ort bewegen.

In einer bevorzugten Ausführungsform sind die erste Hülse und die zweite Hülse in der Längsrichtung hintereinander angeordnet, wobei sie in der Längsrichtung relativ zueinander unverschieblich miteinander verbunden sind.

Vorzugsweise sind die erste Hülse und die zweite Hülse gemeinsam relativ zum Katheterschlauch in Längsrichtung verschiebbar.

Damit die Katheterspitze beim Durchschieben durch Körperkanäle auch um enge Kurven geschoben werden kann, ohne das umliegende Gewebe zu traumatisieren, weist die Katheterspitze vorzugsweise mindestens einen Biegebereich auf, so dass sie quer zur Längsrichtung biegbar ist. Dies ermöglicht es, die Katheterspitze relativ lang auszubilden und insbesondere die zwei Druckkammern hinter einander anzuordnen.

In einer bevorzugten Ausführungsform ist am distalen Ende des Katheterschlauchs ein erster Katheterkolben angeordnet, welcher ein distales Ende der ersten Druckkammer bildet, wobei die erste Hülse dichtend gleitend relativ zu diesem ersten Katheterkolben in der Längsrichtung verschiebbar ist, wobei die erste Druckkammer einen ersten Hülsenboden aufweist, welcher ein proximales Ende der ersten Druckkammer bildet, wobei der erste Hülsenboden fest mit der ersten Hülse verbunden ist und dichtend gleitend um den Katheterschlauch angeordnet ist, wobei am Katheterschlauch ein zweiter Katheterkolben angeordnet ist, welcher ein proximales Ende der zweiten Druckkammer bildet, wobei die zweite Hülse dichtend gleitend relativ zu diesem zweiten Katheterkolben in der Längsrichtung verschiebbar ist, wobei die zweite Druckkammer einen zweiten Hülsenboden aufweist, welcher ein distales Ende der zweiten Druckkammer bildet und wobei der zweite Hülsenboden fest mit der zweiten Hülse verbunden ist und dichtend gleitend um den Katheterschlauch angeordnet ist.

Diese Ausführungsform lässt sich sehr schmal im Durchmesser ausbilden.

In einer anderen bevorzugten Ausführungsform ist die zweite Hülse in der ersten Hülse und relativ zu dieser verschiebbar angeordnet. Vorzugsweise weist in dieser Ausführungsform der Katheterschlauch einen äusseren Schlauchteil und einen darin angeordneten inneren Schlauchteil auf, wobei der äussere Schlauchteil relativ zum inneren Schlauchteil in der Längsrichtung verschiebbar ist, wobei die erste Hülse fest mit dem äusseren Schlauchteil verbunden ist und dichtend gleitend gegenüber dem inneren Schlauchteil in der Längsrichtung verschiebbar ist, wobei ein Katheterkolben vorhanden ist, welcher fest mit dem inneren Schlauchteil verbunden ist und welcher dichtend relativ zur inneren Hülse in der Längsrichtung verschiebbar ist, wobei die zweite Hülse an ihrem proximalen Ende mit einem Hülsenboden verschlossen ist, welcher fest mit ihr verbunden ist und welcher dichtend gleitend relativ zur ersten Hülse in der Längsrichtung verschiebbar ist, wobei die erste Druckkammer zwischen dem Hülsenboden und dem Katheterkolben ausgebildet ist und die zweite Druckkammer zwischen einem proximalen Ende der ersten Hülse und dem Hülsenboden ausgebildet ist. Diese Ausführungsform weist eine relativ kleine Länge auf.

In beiden und weiteren Ausführungsformen bildet vorzugsweise der dem distalen Ende des Katheterschlauchs am nächsten liegende Katheterkolben ein distales Ende der Stentkammer. Das heisst, die Stentkammer ist vor den zwei Druckkammern angeordnet.

In beiden und weiteren Ausführungsformen weist die Katheterspitze an ihrem distalen Ende einen Katheterkopf in Form von Federblättern auf, wobei die Federblätter blüten- oder knospenförmig zueinander geneigt sind und eine Öffnung zur Stentkammer mindestens teilweise verschliessen. Vorzugsweise weist jedes Federblatt eine nach innen gerichtete Ausbuchtung auf. Es lassen sich auch andere Formen von atraumatischen Katheterköpfen verwenden.

Das Zuführungssystem weist vorzugsweise ein Manipulationsteil auf, welches am proximalen Ende des Katheterschlauchs angeordnet ist, wobei mittels des Manipulationsteils der Stent am vorgegebenen Ort im Körpergefäss aus der Stentkammer freisetzbar und in diese wieder rückführbar ist.

In einer bevorzugten Ausführungsform weist das Manipulationsteil eine Hydraulikeinrichtung mit einer ersten Fluidkammer mit einem ersten Kolben und einer zweiten Fluidkammer mit einem zweiten Kolben auf, wobei ein proximales Ende des ersten Fluidlumens mit der ersten Fluidkammer verbunden ist und ein proximales Ende des zweiten Fluidlumens mit der zweiten Fluidkammer verbunden ist. Vorzugsweise sind dabei der erste Kolben mittels eines Betätigungshebels und der zweite Kolben mittels eines Drehknopfes betätigbar.

Vorzugsweise ist der Katheterkopf möglichst atraumatisch. Ein derartiger Katheterkopf weist vorzugsweise Federblätter auf, die blüten- oder knospenförmig zueinander geneigt sind und sich durch Druck von innen nach aussen öffnen.

### öffnen.

Vorzugsweise federn die Federblätter nach Freisetzung eines Stents selbsttätig in ihre Knospenform zurück.

Um das Freisetzen eines Stents und auch eine allfällige erneute Umhüllung zu erleichtern, weisen die Federblätter vorzugsweise nach innen gerichtete Ausbuchtungen auf.

Das Traumatisierungsrisiko lässt sich noch weiter vermindern, wenn die Federblätter gemeinsam von einem flexiblen Schutzschlauch umgeben sind, welcher sich beim Öffnen der Federblättern nach aussen dehnt und elastisch genug ist, um beim erneuten Schliessen der Federblätter wieder an diese anzuliegen.

Dieser Katheterkopf eignet sich für diverse Anwendungen, insbesondere jedoch zur Verwendung in einem in diesem Text beschriebenen Zuführungssystem.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine perspektivische Darstellung eines erfindungsgemässen Stentapplikators in einer ersten Ausführungsform;
- Figur 2: einen Längsschnitt durch den Stentapplikator gemäss Figur 1 mit teilweise freigegebem Stent;
- Figur 3: einen Längsschnitt durch einzelne Bereiche des Stentapplikators gemäss Figur 1 mit eingehülltem Stent;
- Figur 4: einen Längsschnitt durch einzelne Bereiche des Stentapplikators gemäss Figur 1 mit teilweise freigegebenem Stent;
- Figur 5: eine vergrösserte Darstellung des vorderen Bereichs gemäss Figur 3;
- Figur 6: eine vergrösserte Darstellung des vorderen Bereichs gemäss Figur 4;
- Figur 7: eine perspektivische Darstellung eines erfindungsgemässen Stentapplikators in einer zweiten Ausführungsform;
- Figur 8: einen Längsschnitt durch einen vorderen Bereich des Stentapplikators mit eingehülltem Stent gemäss Figur 7;
- Figur 9: einen Längsschnitt durch einen vorderen Bereich des Stentapplikators gemäss Figur 7 mit vollständig umhülltem Stent;
- Figur 10: den Stentapplikator gemäss Figur 9 mit teilweise freigegebenem Stent;
- Figur 11: den Stentapplikator gemäss Figur 9 mit noch weiter freigegebenem Stent;
- Figur 12: den Stentapplikator gemäss Figur 9 mit teilweise erneut umhüllten Stent;
- Figur 13: den Stentapplikator gemäss Figur 9 mit noch weiter umhüllten Stent, analog zu Figur 7
- Figur 14: den Stentapplikator gemäss Figur 9 beim erneuten Umhüllen der inneren Hülse und
- Figur 15: den Stentapplikator gemäss Figur 9 in seiner erneuten Ausgangsstellung.

### BESCHREIBUNG BEVORZUGTER AUSFÜHRUNGSFORMEN

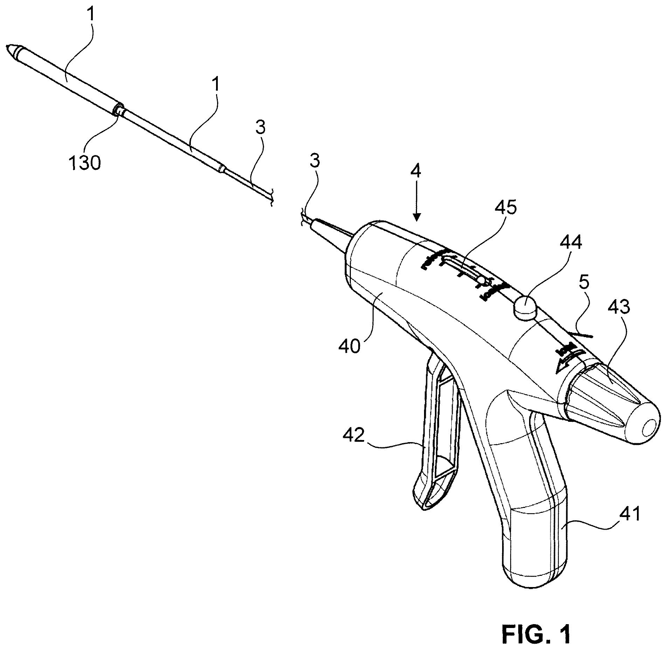

In den Figuren 1 bis 6 ist ein erstes Ausführungsbeispiel eines erfindungsgemässen Stentapplikators dargestellt. Er weist ein Manipulationsbesteck in Form eines pistolenförmigen, steifen Handteils 4 auf. Dieses Handteil 4 weist einen Grundkörper 40 mit einem daran angeformten Handgriff 41 zum Halten des Bestecks in der Hand des Arztes oder Anwenders auf. Ein Betätigungshebel 42 lässt sich mit den Fingern der Hand, welche den Handgriff 41 hält, zum Handgriff 41 hin ziehen.

Am Handteil 4 ist ein flexibler Katheterschlauch 3 befestigt. Vorzugsweise ist er lösbar damit verbunden. Er kann jedoch auch fest bzw. nicht zerstörungsfrei lösbar mit dem Handteil 4 verbunden sein. Der Katheterschlauch 3 ist aus einem biegsamen Material, insbesondere aus Pebax gefertigt. Er weist eine an den Anwendungsbereich des Stentapplikators angepasste Länge auf. In den Figuren ist er in seiner Länge unterbrochen dargestellt.

Der Katheterschlauch 3 endet an seinem freien, dem Handteil 4 abgewandten Ende in einer Katheterspitze 1. Diese Katheterspitze 1 ist in diesem Beispiel relativ lang ausgebildet, wie dies in Figur 1 erkennbar ist. Die Katheterspitze 1 weist deshalb vorzugsweise einen oder mehrere Biegebereiche 160 auf, an welchen sie gebogen werden kann. Dadurch lässt sich die Katheterspitze 1 auch um enge Kurven durch Körperkanäle des Patienten führen.

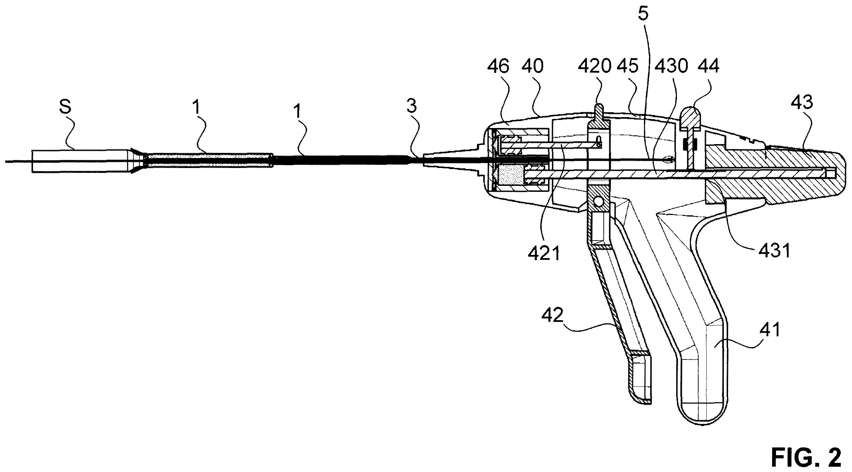

In der Katheterspitze 1 ist ein Stent S angeordnet, welcher mittels des Handteils 4 freigegeben werden kann. In Figur 1 ist der Stent S vollständig in die Katheterspitze 1 aufgenommen und deshalb nicht sichtbar. In Figur 2 ist er praktisch vollständig freigegeben, wobei er mit seinem hinteren, dem Handteil 4 zugewandten Ende noch in der Katheterspitze 1 gehalten ist.

Der Stent S ist hier schematisch dargestellt. Vorzugsweise handelt es sich um einen selbstexpandierenden Stent S. Er kann eine beliebige Form aufweisen. Beispielsweise kann er aus einem federnden rohrförmigen Gittergeflecht bestehen. Diverse andere Formen sind aus dem Stand der Technik bekannt. Der Stent kann beispielsweise auch mit einem Graft versehen sein, wobei der Graft Klappen oder keine Klappen aufweisen kann.

Der Stent ist vorzugsweise in der Katheterspitze gehalten, beispielweise ist er in, in diesem Ausführungsbeispiel nicht dargestellten, Haken lösbar eingehängt. Es können auch andere Arten von Rückhaltemitteln verwendet werden.

Wie in Figur 2 erkennbar ist, wirkt der Betätigungshebel 42 auf eine erste Kolbenstange 421. Diese Kolbenstange 421 ist Teil einer Hydraulikeinrichtung 46, welche im Handteil 4 angeordnet ist. Am oberen Ende des Betätigungshebels 42 ist eine vorstehende Nase ausgebildet, welche das Gehäuse des Grundkörpers 40 nach aussen durchdringt und sich als Anzeigestift 420 entlang eines Anzeigeschlitzes 45 in Abhängigkeit der Bewegung des Betätigungshebels 42 bewegt. Dadurch kann von aussen, wie in Figur 1 erkennbar ist, die aktuelle Funktion des Handteils 4 abgelesen werden.

Das hintere Ende des Handteils 4 ist mit einem Drehknopf 43 versehen. Dieser Drehknopf 43 dreht eine zweite Kolbenstange 430 der Hydraulikeinrichtung 46. Diese zweite Kolbenstange 430 ist mit einem Gewinde 431 versehen bzw. sie ist über einen Abschnitt als umlaufende Zahnstange ausgebildet. Auf diesen Abschnitt wirkt ein Mitnehmer 44, welcher in das Gewinde 431 bzw. die Zahnstange eingreift und so die Kolbenstange nach vorne in Richtung Katheterspitze 1 schiebt.

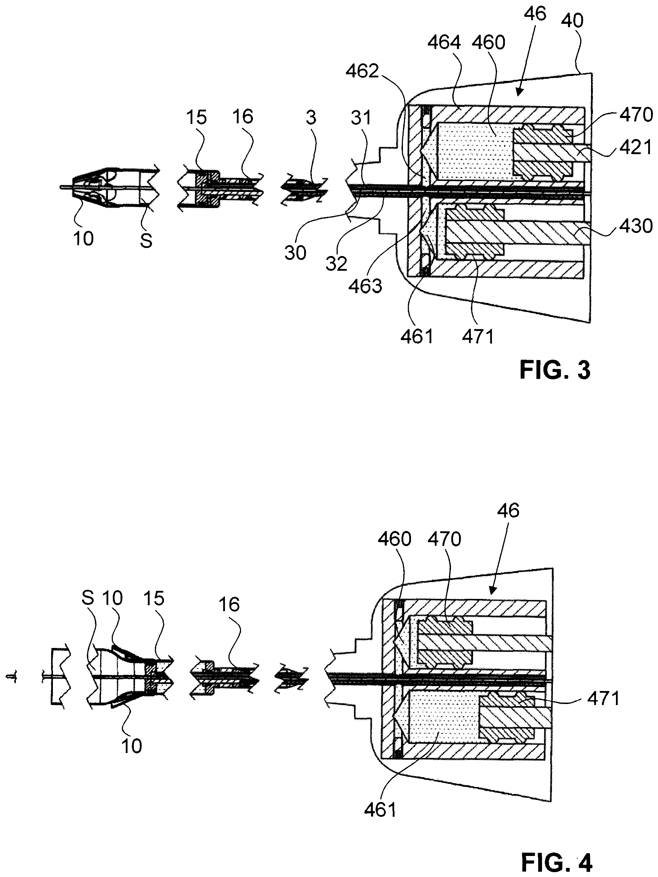

Die Hydraulikeinrichtung 46 weist, wie dies in Figur 3 gut erkennbar ist, eine erste und eine zweite Fluidkammer 460, 461 auf, welche nebeneinander angeordnet sind. Die erste Kolbenstange 421 endet mit einem ersten Kolben 470 in der ersten Fluidkammer 460; die zweite Kolbenstange 430 endet mit einem zweiten Kolben 471 in der zweiten Fluidkammer 461. Beide Kolben 470, 471 sind mittels der zugehörigen Kolbenstange 421, 430 in der jeweiligen Fluidkammer 460, 461 in Längsrichtung verschiebbar, wobei sie gegenüber einem äusseren Gehäuse 464 der Hydraulikeinrichtung 46 dichtend gelagert sind.

Im Gehäuse 464 der Hydraulikeinrichtung 46 führt ein erster Fluidkanal 462 von der ersten Fluidkammer 460 zu einer ersten Eingangsöffnung eines ersten Fluidzuführungslumens 31 des Katheterschlauchs 3. Ein zweiter Fluidkanal 463 führt von der zweiten Fluidkammer 461 zu einer ersten Eingangsöffnung eines zweiten Fluidzuführungslumens 32 des Katheterschlauchs 3.

Der Katheterschlauch 3 weist ferner ein Zentrallumen 30 auf, durch welches ein Führungsdraht 5 vom Handteil 4 bis zur Katheterspitze 1 und darüber hinaus geführt werden kann. Wie in Figur 1 erkennbar ist, ist der Führungsdraht mit seinem proximalen Ende aus dem Handteil 4 herausgeführt, so dass er vom Anwender in der Hand gehalten werden kann.

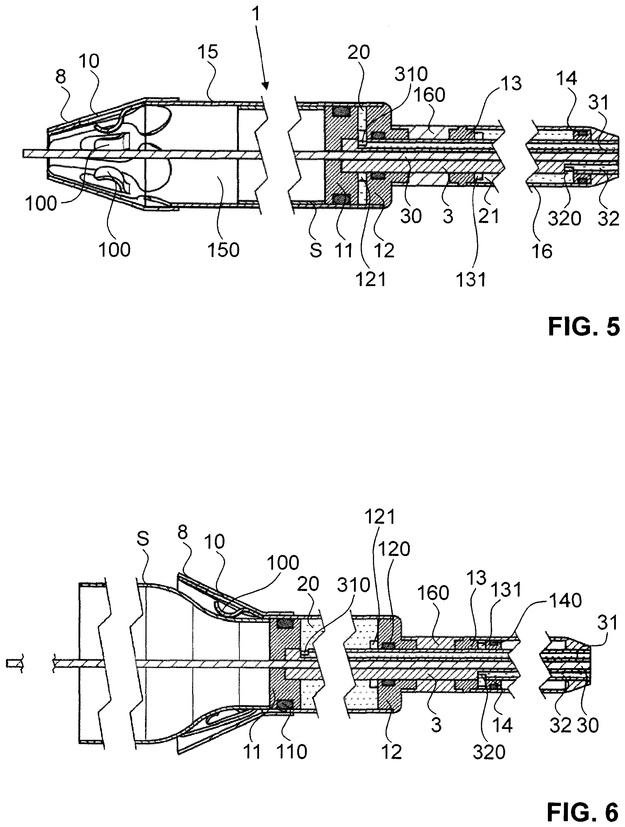

Die Katheterspitze 1 ist in Figur 5 gut erkennbar. Sie weist zwei hintereinander angeordnete Hülsen 15, 16 auf, welche vorzugsweise unterschiedliche Aussendurchmesser aufweisen. Die zwei Hülsen 15, 16 sind vorzugsweise unelastisch, aber biegsam ausgebildet. Sie bestehen vorzugsweise aus einem faserverstärkten Kunststoff, z.B. aus verstärktem Pebax. Die erste Hülse 15 bildet eine Stentkammer 150 zur Aufnahme des Stents S. Der Innendurchmesser der ersten Hülse 15 ist so bemessen, dass der Stent S im radial komprimierten Zustand in der Stentkammer 150 gehalten werden kann.

Das vordere, dem Handteil 4 abgewandte Ende der ersten Hülse 15 geht in Federblätter 10 über. Sie bilden einen Kopfteil des Katheters. Diese sind vorzugsweise einstückig mit der ersten Hülse 15 ausgebildet. Diese Federblätter 10 verschliessen das vordere freie Ende der ersten Hülse 15 blütenförmig. Jedes Federblatt 10 weist vorzugsweise eine nach innen gerichtete Ausbuchtung 100 auf.

Die erste Hülse 15 ist über einen ersten Hülsenboden 12 dicht und fest mit der zweiten Hülse 16 verbunden. Die zweite Hülse 16 weist vorzugsweise einen geringeren Aussendurchmesser auf als die erste Hülse 15. Im Innern der zweiten Hülse 16, vorzugsweise in ihrem in Längsrichtung gesehen mittleren Bereich, ist ein zweiter Hülsenboden 13 lagefixiert angeordnet. Dieser zweite Hülsenboden 13 unterteilt den Innenraum der zweiten Hülse in zwei Kammern. Dieser Hülsenboden 13 durchsetzt vorzugsweise die Wandung der zweiten Hülse 16.

Der zweite Hülsenboden 13 ist beabstandet zum ersten Hülsenboden 12 angeordnet. Der dazwischen liegende Bereich ist als Biegebereich 160 ausgebildet. Er ist vorzugsweise aus einem elastischen Material, beispielsweise Silikon, gefertigt.

Der erste und zweite Hülsenboden 12, 13, können auch direkt aneinander angrenzen bzw. durch ein gemeinsames, insbesondere einstückiges Bauteil gebildet sein. Vorzugsweise sind sie in diesen Varianten biegsam ausgebildet, so dass sie selber das Gelenk bzw. den Biegebereich 160 bilden können.

Der Katheterschlauch 3 durchsetzt die zweite Hülse 16 und endet in der ersten Hülse 15. An seinem freien Ende ist ein erster Katheterkolben 11 fest angeordnet. Der erste Katheterkolben 11 befindet sich im Innern der ersten Hülse 15 und bildet eine handteilseitige Wand der Stentkammer 150. Der erste Katheterkolben 11 ist relativ zur ersten Hülse 15 verschiebbar in dieser gehalten. Dabei ist er gegenüber der Innenwand der ersten Hülse 15 mit einem ersten Dichtring 110 gedichtet.

Der in seiner Grösse veränderbare Raum zwischen dem ersten Katheterkolben 11 und dem ersten Hülsenboden 12 bildet eine erste Druckkammer 20, deren Funktionsweise später erläutert wird. Das erste Fluidlumen 31 des Katheterschlauchs 3 endet in dieser ersten Druckkammer 20. Die in diese erste Druckkammer 20 führende Lumenöffnung ist mit dem Bezugszeichen 310 versehen. Dadurch ist eine Leitungsverbindung von der ersten Fluidkammer 460 im Handteil 4 über den Katheterschlauch 3 bis zur ersten Druckkammer 20 geschaffen. Dieses System ist mit einem ersten Fluid, vorzugsweise einer Flüssigkeit gefüllt. Als Flüssigkeit eignen sich insbesondere eine Kochsalzlösung, ein Kontrastmittel oder andere biokompatible Flüssigkeiten. Die Flüssigkeit ist vorzugsweise hochviskos, um die Dichtheit des Systems zu gewährleisten.

Am Katheterschlauch 3 ist ferner ein zweiter Katheterkolben 14 fest angeordnet. Er ist innerhalb der zweiten Hülse 16 in Längsrichtung verschiebbar gehalten, wobei auch er gegenüber der Innenwand der zweiten Hülse 16 mit mindestens einem zweiten Dichtring 140 gedichtet ist. Zwischen diesem zweiten Katheterkolben 14 und dem zweiten Hülsenboden 13 ist eine zweite Druckkammer 21 gebildet. Das zweite Fluidlumen 32 des Katheterschlauchs endet in einer zweiten Lumenöffnung 320, welche in diese zweite Druckkammer 21 führt. Somit ist über den Katheterschlauch 3 eine Leitung zwischen der zweiten Fluidkammer 461 im Handteil 4 und der zweiten Druckkammer 21 gebildet. Dieses System ist ebenfalls mit einem zweiten Fluid, vorzugsweise einer Flüssigkeit, insbesondere einer hochviskosen Flüssigkeit gefüllt. Es lassen sich dieselben Beispiele wie oben angeben. Vorzugsweise sind beide Systeme mit derselben Art Fluid gefüllt. Die Fluide der zwei Systeme vermischen sich jedoch nicht miteinander.

Der Katheterschlauch 3 durchsetzt den ersten und den zweiten Hülsenboden 12, 13 und ist relativ zu diesen in Längsrichtung verschiebbar. Der ersten Hülsenboden 12 ist gegenüber dem äusseren Mantel des Katheterschlauchs 3 mit einem Dichtring 120 gedichtet.

Die genauere Wirkungsweise der Hydraulikeinrichtung 46 mit dem doppeltwirkenden Zylinder ist in der Zusammenschau der Figuren 3 bis 6 erkennbar. In den Figuren 3 und 5 ist der Stent S in der Katheterspitze angeordnet und vollständig umhüllt. Der erste Kolben 470 befindet sich in der zurückgezogenen Position und der in Figur 2 sichtbare Anzeigestift 421 befindet sich in der hinteren Lage, d.h. anders als in Figur 2 dargestellt. Die erste Fluidkammer 460, gebildet durch den ersten Zylinder, ist mit dem ersten Fluid gefüllt. Das Volumen der ersten Druckkammer 20 ist auf ein Minimum reduziert. Vorzugsweise ist der erste Hülsenboden 12 bis auf einen Abstandhalter 121 in der ersten Druckkammer 20 dem ersten Katheterkolben 11 angenähert.

Der zweite Kolben 471 befindet sich in dieser Position am vorderen, katheterseitigen Anschlag der zweiten Fluidkammer 461. Das zweite Fluid befindet sich vor allem in der zweiten Druckkammer 21. Diese ist vorzugsweise auf ihr Maximum ausgedehnt, das heisst, der zweite Katheterkolben 14 befindet sich am handteilseitigen Ende der zweiten Hülse 16 im maximalen Abstand zum zweiten Hülsenboden 13.

Diese Situation wird als "geladen" bezeichnet. Der Betätigungshebel 42 des Handteils 4 ist nicht betätigt.

Wird nun der Betätigungshebel 42 betätigt, d.h. zum Handgriff 41 hin gezogen, so drückt die erste Kolbenstange 421 den ersten Kolben 470 im Zylinder nach vorne und das erste Fluid wird in die erste Druckkammer 20 geleitet. Der Anzeigestift 420 wird zusammen mit der ersten Betätigungsstange 421 nach vorne geschoben und zeigt nun, wie in Figur 2 dargestellt, die "freigesetzte" Situation des Stents S an. Diese Situation ist in den Figuren 4 und 6 dargestellt. Der verschiebbar um den Katheterschlauch 3 angeordnete erste Hülsenboden 12 wird nach hinten in Richtung Handteil 4 gedrückt und zieht bzw. stösst die erste und zweite Hülse 15, 16 nach hinten.

Da der Stent S auf dem ersten Katheterkolben 11 aufliegt, stossen die Federblätter 10 an diesen an und öffnen sich nach aussen. Sie gleiten über den Stent S hinweg, so dass dieser freigesetzt wird. Der befreite Bereich des Stents S entfaltet sich daraufhin, vorzugsweise selbsttätig, das Material des Stents S entspannt sich und der Stent S fixiert sich vorzugsweise durch eine Restkomprimierung respektive Restvorspannung in der anatomischen Struktur der Einsatzregion.

Die zweite Hülse 16 gleitet über den zweiten Katheterkolben 14 und der zweite Hülsenboden 13 nähert sich so diesem Kolben 14 bis auf einen Abstandhalter 131 an. Dadurch wird das Volumen der zweiten Druckkammer 21 verkleinert und das zweite Fluid wird in die zweite Fluidkammer 461 im Handteil 4 zurückgedrückt. Der zweite Kolben 471 wird nach hinten bewegt. Hierfür ist der Mitnehmer 44 im Handteil 4 von der zweiten Betätigungsstange 430 beabstandet, d.h. er ist leicht aus dem Grundkörper 40 herausgezogen und gibt somit die Zahnstange 431 frei.

Soll nun der freigesetzte oder teilweise freigesetzte Stent S wieder in die Katheterspitze 1 zurückgeholt und neu platziert oder ganz aus dem Körper des Patienten entfernt werden, so erfolgt dies mittels des Drehknopfes 43 des Handteils 4. Hierfür ist der Mitnehmer 44 im Eingriff mit dem Gewinde 431 der zweiten Betätigungsstange 430. Wird der Drehknopf 43 von Hand gedreht, so verschiebt sich die zweite Betätigungsstange 430 nach vorne. Der zweite Kolben 471 drückt wieder das zweite Fluid in die zweite Druckkammer 21 der Katheterspitze 1 und die zwei Hülsen 15, 16 werden wieder nach vorne geschoben. Die Federblätter 10 schieben sich über den Stent und fangen diesen wieder ein. Falls notwendig, öffnen sie sich hierzu.

Dabei wird wiederum das erste Fluid aus der ersten Druckkammer 20 in die erste Fluidkammer 460 zurück geleitet, bis die "geladene" Situation gemäss den Figuren 3 und 5 wieder erreicht ist. Sobald der Stent S wieder mehrheitlich oder ganz umhüllt ist, kann die Position innerhalb des Körpers des Patienten wunschgemäss korrigiert werden und der Stent S anschliessend wieder frei gesetzt werden.

Da für dieses erneute Umhüllen des Stents S hohe Kompressionskräfte nötig sind, wurde zur Herabsetzung der Kräfte eine blumenartige Struktur, hier die Federblätter 10 geschaffen. Es sind vorzugsweise zwei, drei, vier, fünf oder mehr Federblätter 10 vorhanden. Sie sind vorzugsweise relativ zur ersten Hülse 15 federnd ausgebildet und nehmen selbsttätig ihre geschlossene Position gemäss den Figuren 3 und 5 ein. Sie bestehen vorzugsweise aus Nitinol. Beim Öffnen entsteht wie bei einer sich öffnenden Blume ein Trichter. Dieser Trichter erleichtert das Herausgleiten, jedoch auch das erneute Umhüllen, da der Stent nicht über eine scharfe Kante in seine komprimierte Form zurückgedrängt wird, sondern über diese Trichtergeometrie.

Wenn der Stent S korrekt platziert ist, kann das Zuführungssystem aus dem Patientenkörper herausgezogen werden. Die Federblätter 10 an der Katheterspitze 1 vermindern dabei das Traumatisierungsrisiko. Da sie nach Freisetzung des Stents S selbsttätig in ihre geschlossene Position zurückfedern, bilden sie beim Herausziehen des Systems keinen erhöhten Widerstand.

Vorzugsweise ist die durch die Federblätter 10 gebildete geschlossene Blütenstruktur mit einem elastischen Schutzschlauch 8 überzogen. Der Schutzschlauch ist vorzugsweise aus Silikon gefertigt. Dieser Schutzschlauch 8 ist proximal und distal offen ausgebildet und vorzugsweise proximal eingeklebt. Es lässt sich auch ein Schrumpfschlauch als Schutzschlauch 8 verwenden. Der Schutzschlauch 8 umgibt die Federblätter 10 vollständig. Er lässt sich beim Öffnen der Federblätter 10 genügend dehnen, damit der Stent S freigesetzt werden kann. Er ist zudem elastisch genug, damit er sich beim erneuten Schliessen der Federblätter 10 ebenfalls zusammenzieht und so diese wieder mindestens annähernd fest umgibt.

Die Anordnung der Druckkammern 20, 21 der Katheterspitze in Längsrichtung hintereinander hat den Vorteil, dass die Katheterspitze bzw. der Katheterkopf einen relativ kleinen Aussendurchmesser aufweisen kann. Die Grösse des Stents S und die Wandstärke der Hülse definieren diesen Aussendurchmesser.

Es sind Variationen dieser Ausführungsform möglich. So kann z.B. anstelle von zwei Hülsen 15, 16 auch eine einzige ein- oder mehrstückige, vorzugsweise biegsame Hülse verwendet werden, in welcher die zwei Kolben und die zwei Böden bzw. der eine Boden angeordnet sind.

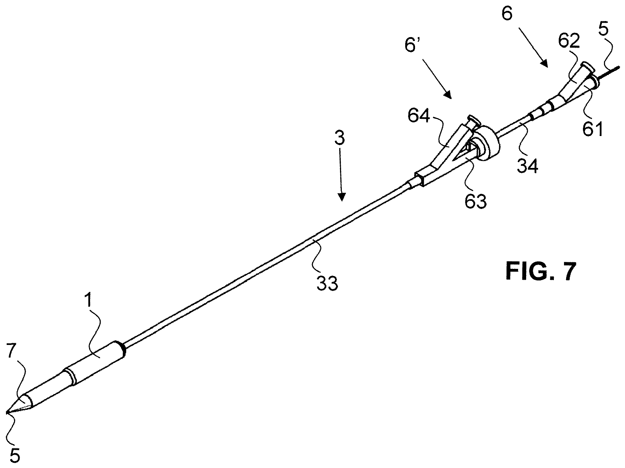

In den Figuren 7 bis 15 ist eine zweite Ausführungsform des erfindungsgemässen Zuführungssystems dargestellt. Im Gegensatz zum ersten Ausführungsbeispiel sind nun die zwei Druckkammern konzentrisch zueinander angeordnet. Dies hat den Vorteil, dass die Katheterspitze kürzer ausgebildet werden kann und somit einfacher um enge Kurven in den Körperkanälen durchgeführt werden kann.

Das Zuführsystem weist eine Katheterspitze 1 mit einer äusseren Hülse 17 auf. Diese Katheterspitze 1, genauer die äussere Hülse 17, ist am vorderen, distalen Ende mit einem Katheterkopf 7 verschliessbar. Die äussere Hülse 17 ist vorzugsweise unelastisch jedoch biegsam ausgebildet. Vorzugsweise ist sie aus einem faserverstärkten Kunststoff, z.B. aus verstärktem Pebax.

Wie in Figur 7 erkennbar, ist ein flexibler Katheterschlauch 3, vorzugsweise aus Pebax, mit der Katheterspitze 1 fest verbunden. An seinem hinteren, d.h. proximalen Ende ist der Katheterschlauch mit einer Konnektoreinheit, vorzugsweise mit zwei hinter einander angeordneten Y-Konnektoren 6, 6', verbunden. Ein Führungsdraht 5 durchsetzt die Konnektoreinheit 6, 6', den Katheterschlauch 3, die Katheterspitze 1 wie auch den Katheterkopf 7, wobei er aus einem ersten Anschlussteil 61 der Konnektoreinheit, genauer des ersten Y-Konnektors 6, herausragt. Der Führungsdraht 5 dient üblicherweise der Einführung des Katheterschlauchs 3 in einen Körperkanal des Patienten.

Ein zweites Anschlussteils 62 des ersten Y-Konnektors 6 dient dazu, ein Fluid in einen inneren Schlauchteil 34 eines Katheterschlauchs 3 bis zur Katheterspitze 1 einzubringen. Dies erfolgt beispielsweise durch eine manuell oder motorisch betriebene Spritze. Geeignete Fluide wurden bereits weiter oben angegeben.

Der innere Schlauchteil 34 durchsetzt den zweiten Y-Konnektor 6', genauer einen dritten Anschlussteil 63 desselben, und ist in Längsrichtung verschiebbar in ihm gehalten. Ein vierter Anschlussteil 64 des zweiten Y-Konnektors 6' dient dazu, ein zweites Fluid durch einen äusseren Schlauchteil 33 des Katheterschlauchs 3 zur Katheterspitze 1 zu bringen. Auch in diesem Beispiel verläuft das zweite Fluid vollständig getrennt vom ersten Fluid. Vorzugsweise besteht es jedoch aus demselben Stoff.

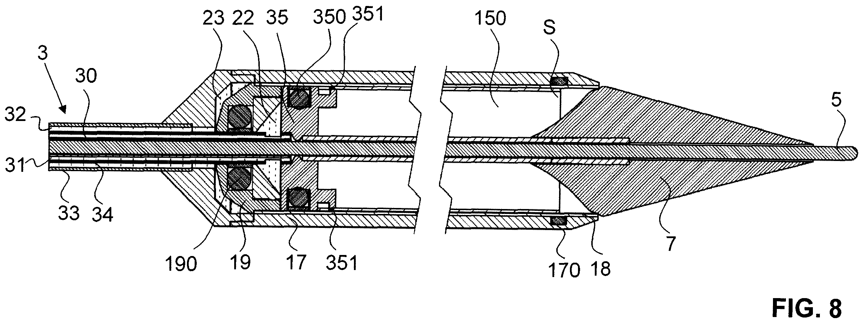

Figur 8 zeigt einen Längsschnitt durch den distalen Bereich des Zuführsystems. Der Katheterschlauch 3 ist auch hier ein mehrlumiger flexibler Schlauch. Er weist einen äusseren Schlauchteil 33 und einen inneren Schlauchteil 34 auf. Der innere Schlauchteil 34 ist in Längsrichtung relativ zum äusseren Schlauchteil 33 verschiebbar.

Der Katheterschlauch 3 weist ein zentrales Lumen 30 zur Aufnahme des relativ zu ihm in Längsrichtung verschiebbaren Führungsdrahts 5 auf. Ferner weist der Schlauch 3 wiederum zwei Fluidlumen 31, 32 auf, welche hier konzentrisch um das Zentrallumen 30 angeordnet sind. Das erste, innere Fluidlumen 31 befindet sich im inneren Schlauchteil 34. Das zweite, äussere Fluidlumen 32 ist durch den Hohlraum zwischen äusserem und inneren Schlauchteil 33, 34 gebildet.

Der äussere Mantel der Katheterspitze 1 ist durch die äussere Hülse 17 gebildet. Am hinteren, proximalen Ende der äussere Hülse 17 ist der äussere Schlauchteil 33 des Katheterschlauchs 3 fest mit dieser äusseren Hülse 17 verbunden. Die äussere Hülse 17 ist an diesem Ende ansonsten geschlossen ausgebildet. Das zweite Fluidlumen 32 endet in einem hinteren, proximalen Hohlraum der äusseren Hülse 17, welcher eine zweite Druckkammer 23 bildet.

In der äusseren Hülse 17 ist eine innere Hülse 18 in Längsrichtung verschiebbar gehalten. Sie ist mit einem oder mehreren Dichtungsringen 170 gegenüber der äusseren Hülse 17 gedichtet. Sie ist an ihrem hinteren proximalen Ende mit einem Hülsenboden 19 geschlossen, welcher fest mit ihr verbunden ist. Dieser Hülsenboden 19 ist vom inneren Schlauchteil 34 des Katheterschlauchs 3 durchsetzt. Er ist in Längsrichtung relativ zum inneren Schlauchteil 34 verschiebbar, wobei er mit einem Dichtring 190 gegenüber dem inneren Schlauchteil 34 gedichtet ist.

Distal zu diesem Hülsenboden 19 durchsetzt der innere Schlauchteil 34 einen Katheterkolben 35, welcher fest mit dem inneren Schlauchteil 34 verbunden ist und relativ zur inneren und somit auch zur äusseren Hülse 18, 17 in Längsrichtung verschiebbar ist. Dieser Katheterkolben 35 ist mit einem Dichtring 350 gegenüber der inneren Hülse 18 gedichtet. Es könnten auch, wie hier dargestellt ist, mehrere Dichtringe, insbesondere zwei Dichtringe 350 vorhanden sein. Der Kolben 35 kann einstückig oder mehrteilig ausgebildet sein.

Zwischen dem Hülsenboden 19 und dem Katheterkolben 35 ist eine erste Druckkammer 22 gebildet. Das erste Fluidlumen 31 endet in dieser ersten Druckkammer 22.

Distal zum Katheterkolben 35 ist in der inneren Hülse 18 eine Stentkammer 150 zur Aufnahme des Stents S ausgebildet. Auch hier ist der Stent S nur schematisch dargestellt. Er kann die üblichen im Stand der Technik bekannten Ausgestaltungsformen aufweisen.

Das distale Ende des Katheterkolbens 35 bildet eine proximale Rückwand der Stentkammer 150. Die distale Wand wird durch den Katheterkopf 7 gebildet, welcher fest mit dem Katheterschlauch 3 verbunden ist und gemeinsam mit diesem verschiebbar ist. Der Katheterkopf 7 ist so bemessen, dass er das distale Ende der inneren Hülse 18 verschliesst.

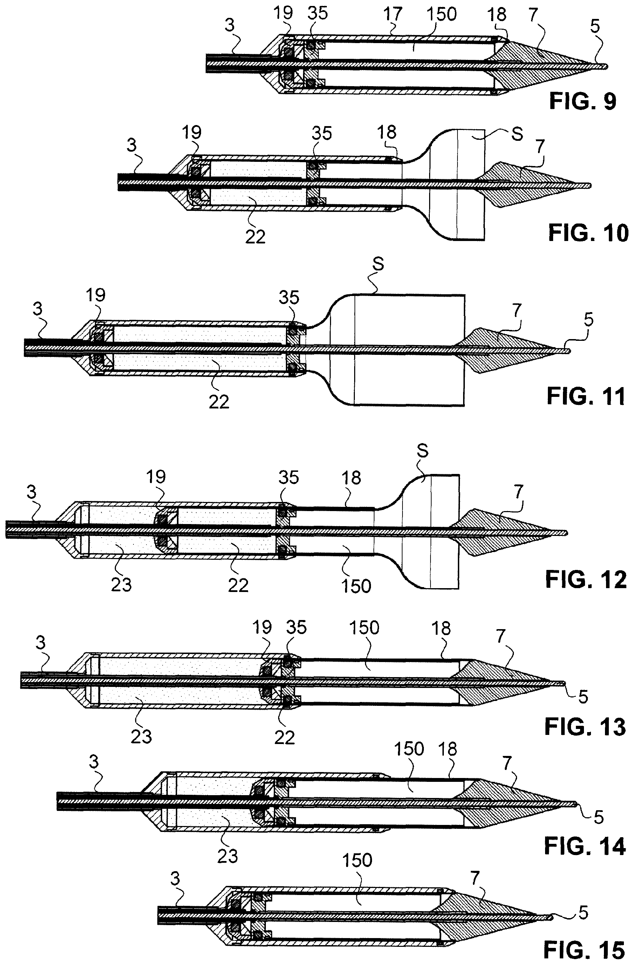

In den Figuren 9 bis 15 ist die Wirkungsweise dieses Zuführsystems erkennbar.

In Figur 9 ist der Stent S in der Stentkammer 150 geladen, wobei die Katheterspitze 1 mit dem Katheterkopf 7 verschlossen ist.

In Figur 10 wird nun über das erste Fluidlumen 31 das erste Fluid in die erste Druckkammer 22 eingeführt. Dadurch bewegt sich die äussere und innere Hülse 17, 18 gemeinsam mit dem äusseren Schlauchteil 33 nach hinten, d.h. in proximale Richtung. Der distale Zugang zur inneren Hülse 18 und auch der Stent S werden freigegeben. Der Stent S kann sich entfalten.

In Figur 11 ist der Zustand dargestellt, in welcher das vordere Ende der Hülsen 17, 18 den Katheterkolben 35 erreicht haben und somit die erste Druckkammer 22 maximal ausgedehnt ist. Der Stent S liegt praktisch frei, ist aber noch an Rückhaltemitteln, hier Haken 351, des Kolbens 35 gehalten.

Soll der Stent S nun in situ neu platziert oder sogar wieder ganz umhüllt werden, so wird nun über das zweite Fluidlumen 32 das zweite Fluid in die zweite Druckkammer 23 eingebracht. Diese dehnt sich zum distalen Ende hin aus und schiebt den Hülsenboden 19 gemeinsam mit der inneren Hülse 18 zum distalen Ende hin und somit aus der äusseren Hülse 17 hinaus. Dies ist in Figur 12 dargestellt. Die innere Hülse 18 schiebt sich über den Stent S und fängt diesen wieder ein.

In Figur 13 hat der Hülsenboden 19 den Kolben 35 erreicht, d.h. die zweite Druckkammer 23 ist maximal ausgedehnt. Das erste Fluid wurde wieder aus der ersten Druckkammer 22 herausgepresst. Die erste Druckkammer 22 hat wieder minimale Grösse erreicht. Das erste Fluid kann auch aktiv aus der ersten Druckkammer 22 entfernt werden. Die innere Hülse 18 hat sich wieder vollständig über den Stent S geschoben und der Katheterkopf 7 verschliesst die innere Hülse 18.

Gemäss Figur 14 lässt sich nun die innere Hülse 18 gemeinsam mit dem Stent S wieder von der äusseren Hülse 17 umhüllen, indem das zweite Fluid aus der zweiten Druckkammer 23 entfernt wird. Das Entfernen des Fluids erfolgt vorzugsweise wiederum über manuell oder motorisch betriebene Spritzen oder Pumpen.

Die Situation gemäss Figur 15 entspricht derjenigen gemäss Figur 9, d.h. die Katheterspitze ist wieder geladen, d.h. der Stent ist umhüllt, und die Druckkammern sind annähernd leer und im Volumen minimiert.

Variationen dieser Ausführungsform sind möglich. So können beispielsweise anstelle des konventionellen Katheterkopfes gemäss den Figuren 7 bis 15 auch die Federblätter und vorzugsweise auch den Schutzschlauch gemäss dem ersten Beispiel an der inneren Hülse angeordnet sein. Des Weiteren kann anstelle des Y-Konnektors auch ein ähnliches Handteil gemäss dem ersten Ausführungsbeispiel verwendet werden.

Dank des erfindungsgemässen Zuführsystems lässt sich ein Stent an wohldefinierter Stelle freisetzen, jedoch auch relativ atraumatisch wieder umhüllen und in situ neu platzieren.

**BEZUGSZEICHENLISTE**

| | | | |
|---|---|---|---|
| 1 | Hülle | 32 | zweites Fluidlumen |
| 10 | Federblatt | 320 | zweite Lumenöffnung |
| 100 | Ausbuchtung | 33 | äusserer Schlauchteil |
| 11 | erster Katheterkolben | 34 | innerer Schlauchteil |
| 110 | erster Dichtring | 35 | Katheterkolben |
| 12 | erster Hülsenboden | 350 | Dichtring |
| 120 | zweiter Dichtring | 351 | Haken |
| 121 | Abstandhalter | | |
| 13 | zweiter Hülsenboden | 4 | Handteil |
| 131 | Abstandhalter | 40 | Grundkörper |
| 14 | zweiter Katheterkolben | 41 | Handgriff |
| 140 | dritter Dichtring | 42 | Betätigungshebel |
| 15 | erste Hülse | 420 | Anzeigestift |
| 150 | Stentkammer | 421 | erste Kolbenstange |
| 16 | zweite Hülse | 43 | Drehknopf |
| 160 | Biegebereich | 430 | zweite Kolbenstange |
| 17 | äussere Hülse | 431 | Zähne |
| 170 | Dichtring | 44 | Arretierung |
| 18 | innere Hülse | 45 | Anzeigeschlitz |
| 19 | Hülsenboden | 46 | Hydraulikeinrichtung |
| 190 | Dichtring | 460 | erste Fluidkammer |
| | | 461 | zweite Fluidkammer |
| 20 | erste Druckkammer | 462 | erster Fluidkanal |
| 21 | zweite Druckkammer | 463 | zweiter Fluidkanal |
| 22 | erste Druckkammer | 464 | Gehäuse |
| 23 | zweite Druckkammer | 470 | erster Kolben |
| | | 471 | zweiter Kolben |
| 3 | Katheter | | |
| 30 | Zentrallumen | 5 | Führungsdraht |
| 31 | erstes Fluidlumen | | |
| 310 | erste Lumenöffnung | 6 | erster Y-Konnektor |
| 6' | zweiter Y-Konnektor | 7 | Katheterkopf |
| 61 | erstes Anschlussteil | | |
| 62 | zweites Anschlussteil | 8 | Schutzschlauch |
| 63 | drittes Anschlussteil | | |
| 64 | viertes Anschlussteil | S | Stent |

## Patentansprüche

1. Zuführungssystem zum Zuführen eines Stents (S) in einen Körper eines Patienten, umfassend:
einen flexiblen Katheterschlauch (3) mit einem proximalen und einem distalen Ende zum Zuführen des Stents (S) an einen vorgegebenen Ort im Körpergefäss und
eine Katheterspitze (1) mit einer Längsrichtung, welche am distalen Ende des Katheterschlauchs (3) angeordnet ist, wobei die Katheterspitze (1) eine Stentkammer (150) zur Aufnahme des Stents (S) aufweist,
wobei die Katheterspitze eine erste Druckkammer (20, 22) aufweist,
wobei im Katheterschlauch (3) ein erster Fluidkanal (31) vom proximalen Ende bis zur ersten Druckkammer (20, 22) der Katheterspitze (1) verläuft und
wobei ein Volumen der ersten Druckkammer (20, 22) durch Zuführen eines ersten Fluids vom ersten Fluidkanal (31) zur ersten Druckkammer (20, 22) vergrösserbar ist und der Stent (S) dadurch aus der Stentkammer (150) am vorgegebenen Ort im Körpergefäss freisetzbar ist,
**dadurch gekennzeichnet,**
**dass** die Katheterspitze (1) eine zweite Druckkammer (21, 23) aufweist,
**dass** im Katheterschlauch (3) ein zweiter Fluidkanal (32) vom proximalen Ende bis zur zweiten Druckkammer (21, 23) verläuft,
**dass** ein Volumen der zweiten Druckkammer (21, 23) durch Zuführen eines zweiten Fluids vom zweiten Fluidkanal (32) zur zweiten Druckkammer (21, 23) vergrösserbar ist, wodurch ein teilweise freigesetzter Stent (S) vom vorgegebenen Ort im Körpergefäss in die Stentkammer (150) rückführbar ist.

2. Zuführungssystem nach Anspruch 1, wobei die Katheterspitze (1) eine erste Hülse (15, 17) aufweist, in welcher die erste Druckkammer (20, 22) angeordnet ist, und eine zweite Hülse (16, 18), in welcher die zweite Druckkammer (21, 23) angeordnet ist.

3. Zuführungssystem nach Anspruch 2, wobei der Katheterschlauch (3) ein Führungslumen (30) zur Durchführung eines Führungsdrahtes (5) aufweist und wobei die erste Hülse (15, 17) und die zweite Hülse (16, 18) relativ zu diesem Führungslumen (30) verschiebbar sind, wobei das Führungslumen (30) in seiner Lage bezüglich des vorgegebenen Ortes unverändert bleibt und sich erste und zweite Hülse (15, 17; 16, 18) relativ zum vorgegebenen Ort bewegen.

4. Zuführungssystem nach einem der Ansprüche 2 bis 3, wobei die erste Hülse (15, 17) und die zweite Hülse (16, 18) gemeinsam relativ zum Katheterschlauch (3) in Längsrichtung verschiebbar sind.

5. Zuführungssystem nach einem der Ansprüche 1 bis 4, wobei die Katheterspitze (1) mindestens einen Biegebereich (160) aufweist, so dass sie quer zur Längsrichtung biegbar ist.

6. Zuführungssystem nach einem der Ansprüche 2 bis 4, wobei die zweite Hülse (18) in der ersten Hülse (17) und relativ zu dieser verschiebbar angeordnet ist.

7. Zuführungssystem nach Anspruch 6,
wobei der Katheterschlauch (3) einen äusseren Schlauchteil (33) und einen darin angeordneten inneren Schlauchteil (34) aufweist,
wobei der äussere Schlauchteil (33) relativ zum inneren Schlauchteil (34) in der Längsrichtung verschiebbar ist,
wobei die erste Hülse (17) fest mit dem äusseren Schlauchteil (33) verbunden ist und dichtend gleitend gegenüber dem inneren Schlauchteil (34) in der Längsrichtung verschiebbar ist,
wobei ein Katheterkolben (35) vorhanden ist, welcher fest mit dem inneren Schlauchteil (34) verbunden ist und welcher dichtend relativ zur zweiten Hülse (18) in der Längsrichtung verschiebbar ist,
wobei die zweite Hülse (18) an ihrem proximalen Ende mit einem Hülsenboden (19) verschlossen ist, welcher fest mit ihr verbunden ist und welcher dichtend gleitend relativ zur ersten Hülse (17) in der Längsrichtung verschiebbar ist,
die erste Druckkammer (22) zwischen dem Hülsenboden (19) und dem Katheterkolben (35) ausgebildet ist und
wobei die zweite Druckkammer (23) zwischen einem proximalen Ende der ersten Hülse (17) und dem Hülsenboden (19) ausgebildet ist.

8. Zuführungssystem nach einem der Ansprüche 6 oder 7, wobei der dem distalen Ende des Katheterschlauchs (3) am nächsten liegende Katheterkolben (11, 35) ein proximales Ende der Stentkammer (150) bildet.

9. Zuführungssystem nach einem der Ansprüche 2 oder 3, wobei die erste Hülse (15) und die zweite Hülse (16) in der Längsrichtung hintereinander angeordnet sind und wobei sie in der Längsrichtung relativ zueinander unverschieblich miteinander verbunden sind.

10. Zuführungssystem nach einem der Ansprüche 2 bis 4,
wobei am distalen Ende des Katheterschlauchs (3) ein erster Katheterkolben (11) angeordnet ist, welcher ein distales Ende der ersten Druckkammer (20) bildet,
wobei die erste Hülse (15) dichtend gleitend relativ zu diesem ersten Katheterkolben (11) in der Längsrichtung verschiebbar ist,
wobei die erste Druckkammer (20) einen ersten Hülsenboden (12) aufweist, welcher ein proximales Ende der ersten Druckkammer (20) bildet,
wobei der erste Hülsenboden (12) fest mit der ersten Hülse (15) verbunden ist und dichtend gleitend um den Katheterschlauch (3) angeordnet ist,
wobei am Katheterschlauch (3) ein zweiter Katheterkolben (14) angeordnet ist, welcher ein proximales Ende der zweiten Druckkammer (21) bildet,
wobei die zweite Hülse (16) dichtend gleitend relativ zu diesem zweiten Katheterkolben (14) in der Längsrichtung verschiebbar ist,
wobei die zweite Druckkammer (21) einen zweiten Hülsenboden (13) aufweist, welcher ein distales Ende der zweiten Druckkammer (21) bildet,
wobei der zweite Hülsenboden (13) fest mit der zweiten Hülse (16) verbunden ist und dichtend gleitend um den Katheterschlauch (3) angeordnet ist.

11. Zuführungssystem nach einem der Ansprüche 1 bis 10, wobei die Katheterspitze (1) an ihrem distalen Ende einen Katheterkopf in Form von Federblättern (10) aufweist, wobei die Federblätter (10) blütenförmig zueinander geneigt sind und eine Öffnung zur Stentkammer (150) mindestens teilweise verschliessen.

12. Zuführungssystem nach Anspruch 11, wobei jedes Federblatt (10) eine nach innen gerichtete Ausbuchtung (100) aufweist.

13. Zuführsystem nach einem der Ansprüche 11 oder 12, wobei ein elastischer Schutzschlauch (8) vorhanden ist, welcher die Federblätter (10) umgibt.

14. Zuführungssystem nach einem der Ansprüche 1 bis 13, wobei es ferner ein Manipulationsteil (4, 6) aufweist, welches am proximalen Ende des Katheterschlauchs (3) angeordnet ist, wobei mittels des Manipulationsteils (4, 6) der Stent (S) am vorgegebenen Ort im Körpergefäss aus der Stentkammer (150) freisetzbar und in diese wieder rückführbar ist.

15. Zuführungssystem nach Anspruch 14, wobei das Manipulationsteil (4) eine Hydraulikeinrichtung (46) mit einer ersten Fluidkammer (460) mit einem ersten Kolben (470) und einer zweiten Fluidkammer (461) mit einem zweiten Kolben (472) aufweist, wobei ein proximales Ende des ersten Fluidlumen (31) mit der ersten Fluidkammer (460) verbunden ist und ein proximales Ende des zweiten Fluidlumen (32) mit der zweiten Fluidkammer (461) verbunden ist.

16. Zuführungssystem nach Anspruch 15, wobei der erste Kolben (470) mittels eines Betätigungshebels (42) und der zweite Kolben (472) mittels eines Drehknopfes (43) betätigbar sind.

## Claims

1. A delivery system for delivering a stent (S) in a body of a patient, comprising:
a flexible catheter tube (3) with a proximal and a distal end for delivering the stent (S) to a predetermined point in the body vessel and
a catheter tip (1) with a longitudinal direction, which is arranged at the distal end of the catheter tube (3), wherein the catheter tip (1) has a stent chamber (150) for receiving the stent (S),
wherein the catheter tip has a first pressure chamber (20, 22),
wherein a first fluid channel (31) extends in the catheter tube (3) from the proximal end to the first pressure chamber (20, 22) of the catheter tip (1) and
wherein a volume of the first pressure chamber (20, 22) is enlargeable by delivering a first fluid from the first fluid channel (31) to the first pressure chamber (20, 22) and, as a result thereof, the stent (S) is releasable from the stent chamber (150) at the predetermined point in the body vessel,
**characterized**
**in that** the catheter tip (1) has a second pressure chamber (21, 23),
**in that** a second fluid channel (32) extends in the catheter tube (3) from the proximal end to the second pressure chamber (21, 23),
**in that** a volume of the second pressure chamber (21, 23) is enlargeable by delivering a second fluid from the second fluid channel (32) to the second pressure chamber (21, 23), as a result of which a partly released stent (S) is retractable into the stent chamber (150) from the predetermined point in the body vessel.

2. The delivery system as claimed in claim 1, wherein the catheter tip (1) has a first sleeve (15, 17), in which the first pressure chamber (20, 22) is arranged, and a second sleeve (16, 18), in which the second pressure chamber (21, 23) is arranged.

3. The delivery system as claimed in claim 2, wherein the catheter tube (3) has a guide lumen (30) for passing a guide wire (5) therethrough and wherein the first sleeve (15, 17) and the second sleeve (16, 18) are displaceable relative to this guide lumen (30), wherein the guide lumen (30) remains unchanged in terms of its location in respect of the predetermined point and the first and second sleeve (15, 17; 16, 18) move relative to the predetermined point.

4. The delivery system as claimed in either of claims 2 and 3, wherein the first sleeve (15, 17) and the second sleeve (16, 18) are displaceable together relative to the catheter tube (3) in the longitudinal direction.

5. The delivery system as claimed in one of claims 1 to 4,
wherein the catheter tip (1) has at least one flexible region (160) such that it is flexible transverse to the longitudinal direction.

6. The delivery system as claimed in one of claims 2 to 4,
wherein the second sleeve (18) is arranged within the first sleeve (17) and in a manner displaceable relative to the latter.

7. The delivery system as claimed in claim 6,
wherein the catheter tube (3) has an outer tube portion (33) and an inner tube portion (34) arranged therein,
wherein the outer tube portion (33) is displaceable in the longitudinal direction relative to the inner tube portion (34),
wherein the first sleeve (17) is securely connected to the outer tube portion (33) and displaceable in the longitudinal direction in a sealing sliding manner in relation to the inner tube portion (34),
wherein a catheter plunger (35) is present, which plunger is securely connected to the inner tube portion (34) and displaceable in the longitudinal direction in a sealing manner relative to the second sleeve (18),
wherein the second sleeve (18) is sealed at the proximal end thereof by a sleeve base (19), which is securely connected to the former and which is displaceable in the longitudinal direction in a sealing sliding manner relative to the first sleeve (17),
the first pressure chamber (22) is embodied between the sleeve base (19) and the catheter plunger (35), and
wherein the second pressure chamber (23) is embodied between a proximal end of the first sleeve (17) and the sleeve base (19).

8. The delivery system as claimed in either of claims 6 and 7, wherein the catheter plunger (11, 35) closest to the distal end of the catheter tube (3) forms a proximal end of the stent chamber (150).

9. The delivery system as claimed in either of claims 2 and 3, wherein the first sleeve (15) and the second sleeve (16) are arranged in succession in the longitudinal direction and wherein, in the longitudinal direction, they are connected to one another in a non-displaceable manner relative to one another.

10. The delivery system as claimed in one of claims 2 to 4,
wherein a first catheter plunger (11) which forms a distal end of the first pressure chamber (20) is arranged at the distal end of the catheter tube (3),
wherein the first sleeve (15) is displaceable in the longitudinal direction in a sealing sliding manner relative to this first catheter plunger (11),
wherein the first pressure chamber (20) has a first sleeve base (12) which forms a proximal end of the first pressure chamber (20),
wherein the first sleeve base (12) is securely connected to the first sleeve (15) and arranged in a sealing sliding manner around the catheter tube (3),
wherein a second catheter plunger (14) which forms a proximal end of the second pressure chamber (21) is arranged on the catheter tube (3), wherein the second sleeve (16) is displaceable in the longitudinal direction in a sealing sliding manner relative to this second catheter plunger (14),wherein the second pressure chamber (21) has a second sleeve base (13) which forms a distal end of the second pressure chamber (21),
wherein the second sleeve base (13) is securely connected to the second sleeve (16) and arranged in a sealing sliding manner around the catheter tube (3).

11. The delivery system as claimed in one of claims 1 to 10,
wherein the catheter tip (1), at the distal end thereof, has a catheter head in the form of spring leaves (10),
wherein the spring leaves (10) are inclined with respect to one another in a petaloid manner and at least partly close an opening to the stent chamber (150).

12. The delivery system as claimed in claim 11, wherein each spring leaf (10) has an inwardly directed protrusion (100).

13. The delivery system as claimed in either of claims 11 and 12, wherein there is an elastic protection tube (8) which surrounds the spring leaves (10).

14. The delivery system as claimed in one of claims 1 to 13,
wherein it furthermore has a manipulation part (4, 6) which is arranged at the proximal end of the catheter tube (3), wherein, by means of the manipulation part (4, 6), the stent (S) can be released from, and returned back into, the stent chamber (150) at the predetermined point in the body vessel.

15. The delivery system as claimed in claim 14, wherein the manipulation part (4) has a hydraulic apparatus (46) with a first fluid chamber (460) with a first plunger (470) and a second fluid chamber (461) with a second plunger (472), wherein a proximal end of the first fluid lumen (31) is connected to the first fluid chamber (460) and a proximal end of the second fluid lumen (32) is connected to the second fluid chamber (461).

16. The delivery system as claimed in claim 15, wherein the first plunger (470) is actuatable by means of an actuation lever (42) and the second plunger (472) is actuatable by means of a rotary knob (43).

## Revendications

1. Système d'alimentation pour l'alimentation d'un stent(S) dans un corps d'un patient, comprenant :
un tube de cathéter flexible (3) avec une extrémité proximale et une extrémité distale pour l'alimentation du stent (S) à un emplacement prédéfini dans le vaisseau du corps et
une pointe de cathéter (1) avec une direction longitudinale qui est disposée à l'extrémité distale du tube de cathéter (3), la pointe de cathéter (1) présentant une chambre de stent (150) pour recevoir le stent(S),
la pointe de cathéter présentant une première chambre de pression (20, 22),
un premier canal fluidique (31) s'étendant dans le tube de cathéter (3) depuis l'extrémité proximale jusqu'à la première chambre de pression (20, 22) de la pointe de cathéter (1) et
un volume de la première chambre de pression (20, 22) pouvant être augmenté par l'alimentation d'un premier fluide depuis le premier canal fluidique (31) jusqu'à la première chambre de pression (20, 22) et le stent (S) pouvant être de ce fait libérée de la chambre de stent (150) à l'emplacement prédéfini dans le vaisseau du corps,
**caractérisé en ce que**
la pointe de cathéter (1) présente une deuxième chambre de pression (21, 23),
un deuxième canal fluidique (32) s'étend dans le tube de cathéter (3) depuis l'extrémité proximale jusqu'à la deuxième chambre de pression (21, 23),
et un volume de la deuxième chambre de pression (21, 23) peut être augmenté par alimentation d'un deuxième fluide depuis le deuxième canal fluidique (32) jusqu'à la deuxième chambre de pression (21, 23), de sorte qu'un stent (S) partiellement libérée puisse être ramenée depuis l'emplacement prédéfini dans le vaisseau du corps jusque dans la chambre de stent (150).

2. Système d'alimentation selon la revendication 1,
dans lequel la pointe de cathéter (1) présente une première douille (15, 17) dans laquelle est disposée la première chambre de pression (20, 22) et une deuxième douille (16, 18) dans laquelle est disposée la deuxième chambre de pression (21, 23).

3. Système d'alimentation selon la revendication 2,
dans lequel le tube de cathéter (3) présente une lumière de guidage (30) pour le passage d'un fil de guidage (5) et la première douille (15, 17) et la deuxième douille (16, 18) pouvant être déplacées par rapport à cette lumière de guidage (30), la position de la lumière de guidage (30) restant inchangée par rapport à l'emplacement prédéfini et la première et la deuxième douille (15, 17 ; 16, 18) se déplaçant par rapport à l'emplacement prédéfini.

4. Système d'alimentation selon l'une quelconque des revendications 2 à 3, dans lequel la première douille (15, 17) et la deuxième douille (16, 18) peuvent être déplacées en commun par rapport au tube de cathéter (3) dans la direction longitudinale.

5. Système d'alimentation selon l'une quelconque des revendications 1 à 4, dans lequel la pointe de cathéter (1) présente au moins une région flexible (160) de telle sorte qu'elle puisse être fléchie transversalement à la direction longitudinale.

6. Système d'alimentation selon l'une quelconque des revendications 2 à 4, dans lequel la deuxième douille (18) est disposée de manière déplaçable dans la première douille (17) et par rapport à celle-ci.

7. Système d'alimentation selon la revendication 6,
dans lequel le tube de cathéter (3) présente une partie de tube extérieure (33) et une partie de tube intérieure (34) disposée dans celle-ci,
la partie de tube extérieure (33) pouvant être déplacée dans la direction longitudinale par rapport à la partie de tube intérieure (34),
la première douille (17) étant connectée fixement à la partie de tube extérieure (33) et pouvant être déplacée dans la direction longitudinale par glissement de manière hermétique par rapport à la partie de tube intérieure (34),
un piston de cathéter (35) étant prévu, lequel est connecté fixement à la partie de tube intérieure (34) et lequel peut être déplacé dans la direction longitudinale de manière hermétique par rapport à la deuxième douille (18),
la deuxième douille (18) étant fermée au niveau de son extrémité proximale par un fond de douille (19) qui est connecté fixement à celle-ci, et qui peut être déplacé de manière hermétique par glissement par rapport à la première douille (17) dans la direction longitudinale,
la première chambre de pression (22) étant réalisée entre le fond de douille (19) et le piston de cathéter (35) et
la deuxième chambre de pression (23) étant réalisée entre une extrémité proximale de la première douille (17) et le fond de douille (19).

8. Système d'alimentation selon l'une quelconque des revendications 6 ou 7, dans lequel le piston de cathéter (11, 35) situé le plus près de l'extrémité distale dudit tube de cathéter (3) forme une extrémité proximale de la chambre de stent (150).

9. Système d'alimentation selon l'une quelconque des revendications 2 ou 3, dans lequel la première douille (15) et la deuxième douille (16) sont disposées l'une derrière l'autre dans la direction longitudinale et dans lequel elles sont connectées l'une à l'autre de manière non déplaçable l'une par rapport à l'autre dans la direction longitudinale.

10. Système d'alimentation selon l'une quelconque des revendications 2 à 4,
dans lequel à l'extrémité distale du tube de cathéter (3) est disposé un premier piston de cathéter (11) qui forme une extrémité distale de la première chambre de pression (20),
la première douille (15) pouvant être déplacée par glissement de manière hermétique par rapport à ce premier piston de cathéter (11) dans la direction longitudinale,
la première chambre de pression (20) présentant un premier fond de douille (12), qui forme une extrémité proximale de la première chambre de pression (20),
le premier fond de douille (12) étant connecté fixement à la première douille (15) et étant disposé de manière à pouvoir coulisser de manière hermétique autour du tube de cathéter (3),
un deuxième piston de cathéter (14) étant disposé au niveau du tube de cathéter (3), lequel forme une extrémité proximale de la deuxième chambre de pression (21),
la deuxième douille (16) pouvant être déplacée dans la direction longitudinale par glissement de manière hermétique par rapport à ce deuxième piston de cathéter (14),
la deuxième chambre de pression (21) présentant un deuxième fond de douille (13) qui forme une extrémité distale de la deuxième chambre de pression (21),
le deuxième fond de douille (13) étant connecté fixement à la deuxième douille (16) et étant disposé de manière à pouvoir coulisser de manière hermétique autour du tube de cathéter (3).

11. Système d'alimentation selon l'une quelconque des revendications 1 à 10, dans lequel la pointe de cathéter (1) présente au niveau de son extrémité distale une tête de cathéter en forme de lames de ressort (10), les lames de ressort (10) étant inclinées les unes par rapport aux autres en forme de fleur et fermant au moins en partie une ouverture vers la chambre de stent (150).

12. Système d'alimentation selon la revendication 11,
dans lequel chaque lame de ressort (10) présente un renflement (100) orienté vers l'intérieur.

13. Système d'alimentation selon l'une quelconque des revendications 11 ou 12, dans lequel un tube de protection élastique (8) est prévu, lequel entoure les lames de ressort (10).

14. Système d'alimentation selon l'une quelconque des revendications 1 à 13, présentant en outre une partie de manipulation (4, 6) qui est disposée au niveau de l'extrémité proximale du tube de cathéter (3), le stent (S) pouvant être libérée de la chambre de stent (150) au moyen de la partie de manipulation (4, 6) à l'emplacement prédéfini dans le vaisseau du corps et pouvant être ramenée à nouveau dans celle-ci.

15. Système d'alimentation selon la revendication 14,
dans lequel la partie de manipulation (4) présente un dispositif hydraulique (46) avec une première chambre de fluide (460) avec un premier piston (470) et une deuxième chambre de fluide (461) avec un deuxième piston (472), une extrémité proximale de la première lumière de fluide (31) étant raccordée à la première chambre de fluide (460) et une extrémité proximale de la deuxième lumière de fluide (32) étant raccordée à la deuxième chambre de fluide (461).

16. Système d'alimentation selon la revendication 15,
dans lequel le premier piston (470) peut être actionné au moyen d'un levier d'actionnement (42) et le deuxième piston (472) peut être actionné au moyen d'un bouton tournant (43).
